(19) **Europäisches Patentamt / European Patent Office / Office européen des brevets**

(11) **EP 3 930 635 B1**

(12) **EUROPEAN PATENT SPECIFICATION**

(45) Date of publication and mention
of the grant of the patent:
**11.09.2024  Bulletin 2024/37**

(21) Application number: **20783668.5**

(22) Date of filing: **25.02.2020**

(51) International Patent Classification (IPC):
**A61F 2/58** *(2006.01)*      **A61F 2/68** *(2006.01)*
**A61F 2/72** *(2006.01)*

(52) Cooperative Patent Classification (CPC):
**A61F 2/586; A61F 2/72; B25J 9/104;
B25J 15/0009;** A61F 2002/5026; A61F 2002/5075;
A61F 2002/5093; A61F 2002/701; B25J 9/1612;
B25J 9/1697

(86) International application number:
**PCT/TH2020/000010**

(87) International publication number:
**WO 2020/204843 (08.10.2020 Gazette 2020/41)**

(54) **DEVICE FOR GRASPING AN OBJECT AND METHOD FOR CONTROLLING THE DEVICE**

VORRICHTUNG ZUM GREIFEN EINES GEGENSTANDES UND VERFAHREN ZUR STEUERUNG DER VORRICHTUNG

DISPOSITIF DE PRÉHENSION D'UN OBJET ET PROCÉDÉ DE COMMANDE DE CE DISPOSITIF

(84) Designated Contracting States:
**AL AT BE BG CH CY CZ DE DK EE ES FI FR GB
GR HR HU IE IS IT LI LT LU LV MC MK MT NL NO
PL PT RO RS SE SI SK SM TR**

(30) Priority:  **28.02.2019  TH 1901001256**

(43) Date of publication of application:
**05.01.2022  Bulletin 2022/01**

(73) Proprietor: **Boonyasurakul, Boonyawee
Bangkok, 10230 (TH)**

(72) Inventors:
• **CHAROENSUK, Warakorn
Bangkok 10800 (TH)**
• **KAIMUK, Panya
Bangkok 10170 (TH)**
• **BOONYASURAKUL, Boonyawee
Bangkok, 10230 (TH)**

(74) Representative: **De Vidal Gonzalez, Maria Ester
Bird & Bird International LLP
Paseo de la Castellana 7, 7º Planta
28046 Madrid (ES)**

(56) References cited:
WO-A1-2004/026540     WO-A2-2008/030419
CN-A- 105 583 839     CN-A- 108 742 957
US-A- 2 549 716     US-A- 3 604 017
US-A- 5 888 213     US-A1- 2006 145 495
US-A1- 2016 074 181     US-A1- 2017 049 583
US-A1- 2017 266 019     US-B1- 6 896 704

## Description

## FIELD OF THE INVENTION

The invention relates to a device for grasping an object, and a method for controlling the device. The device may be a part of a myoelectric prosthetic hand.

## BACKGROUND TO THE INVENTION

The following discussion of the background to the invention is intended to facilitate an understanding of the present invention only. It should be appreciated that the discussion is not an acknowledgement or admission that any of the material referred to was published, known or part of the common general knowledge of the person skilled in the art in any jurisdiction as at the priority date of the invention.

The human hand is one of the most complex human organ. More than 50% of a human's motor cortex is required to control the complex movements of the hand and fingers. Without the hand, it is very difficult to accomplish various tasks in daily life.

The loss of a hand, part of a hand or function associated with a hand leads to inconveniences such as the inability to grasp and manipulate an object, perform gestures to support speech, express oneself, explore the surrounding world, and perform daily living tasks. Apart from these physical problems, a person who lose his/her hand may suffer from psychological problems as well.

Some of the causes that can lead to the loss of a hand are cancer, circulation problem, accident, birth defect, and military combat. Treatment of these upper-limb trauma usually involves amputation of the limb. The amputated level is dependent on the injuries of the limbs and could be partial-hand amputation, wrist disarticulation, transradial amputation (below elbow amputation), elbow disarticulation, transhumeral amputation (above elbow amputation), shoulder disarticulation, and forequarter amputation.

A current solution for treating amputees that have lost their hands, especially those at the transradial amputation level, is to attach a prosthetic hand. The primary purpose of a prosthetic is to mimic the appearance and replace the function of a missing limb. While a single prosthetic that achieves both a natural appearance and extreme functionality would be ideal, most artificial limbs that exist today sacrifice some degree of one for the other. As such, there is a wide spectrum of specialized prosthetics that range from the purely cosmetic (which are inert) to the primarily functional (whose appearance is obviously mechanical).

Functional prosthetics can be broadly categorized into two groups: body-powered and externally-powered prosthetics. Body-powered prosthetics use cables and harnesses strapped to the individual to mechanically maneuver the artificial limb through muscle, shoulder, and arm movement. While they are highly durable, they often sacrifice a natural appearance for moderate functionality and can be fatiguing. An externally-powered artificial limb such as a myoelectric prosthetic are an attempt to solve this physical exertion through using a battery and an electronic system to control movement.

A myoelectric prosthetic hand does not require any unwieldy straps or harnesses to function. Instead, it is custom made to fit and attach to the remaining limb. Once it is attached, the prosthetic uses electronic sensors or electrodes to detect minute muscle, nerve, and electromyography (EMG) activity. It then translates this muscle activity (as triggered by the user) into information that the actuator use to control artificial hand movements. The end result is that the artificial limb moves much like a natural limb, according to the mental stimulus of the user. The user can even control the strength and speed of the limb's movements and grip by varying his or her muscle intensity. In addition to this extreme functionality, the myoelectric artificial limb needs not sacrifice any of its cosmetic appearance. The most advanced versions of these prosthetics are incredibly natural and on par with purely cosmetic limbs.

The myoelectric prosthetic hand on the market commercially are capable of performing various grasp patterns that are commonly used in daily life such as tripod grip, pinch grip, power grip, active index, key grip, finger point, column grip, mouse grip, precision open, precision close, hook grip, finger adduction, open palm, and relax hand. However the current prosthetic hands in the market generally allow users to select at most four grasp patterns for actual operation. This limits the ease of using the prosthetic hand in daily life and reduces the functionality of the prosthetic hand itself.

The requirement of the prosthetic hand in present day is low weight because it will reduce the load that user must receive, thus reduce the fatigue when using the prosthetic hand in long time. Therefore most of the developed prosthetic hand nowadays employ an underactuated mechanism where the number of actuators are less than the number of degrees of freedom of the prosthetic hand, which can help to reduce the overall weight of the prosthetic hand. However, the nature of underactuated fingers and hands creates potentially undesirable properties for robotic grasping and manipulation. This is because an underactuated mechanism creates a negative force which will urge the fingers of the prosthetic hand to move away from an object that the hand is grasping, causing the object to slip out of the grasp of the prosthetic hand. This phenomenon is known in the art as "Ejection". Due to Ejection, the prosthetic hand loses the stability in grasping and hence its functionality.

It is an object of the invention to alleviate one or more of the aforementioned problems or meet one or more of the aforementioned needs, at least in part. Documents US 6 896 704 B1, CN 105 583 839 A and US 2006/145495 A1 show a device for grasping an object.

## SUMMARY OF THE INVENTION

The invention is defined by independent claim 1.

According to a first aspect of the invention, there is a device for grasping an object, the device comprising: an actuator; a proximal element proximal to the actuator; and a tip element distal to the actuator; wherein the actuator provides a primary force on the proximal and tip elements; the proximal and tip elements are coupled by a pivot point connected by a cable to the actuator, and an elastic mechanism acts on the cable to provide a secondary force which displaces the proximal and tip elements towards the object.

The device comprises one or more link elements between the proximal element and the tip element coupled by the at least one pivot point connected by the cable and the elastic mechanism acts on the cable to provide the secondary force to displace the one or more link elements towards the object.

The elastic mechanism comprises one or more springs and wherein each spring has a first end coupled to the cable and a second end coupled to the proximal, link or tip elements. Preferably the spring coupled to the proximal element is connected parallel to the cable. Further preferably the spring coupled to a said link element is connected parallel to the cable.

It is advantageous if the device further comprises a resilient element capable of coupling: a support base of the device to the proximal element; the proximal element to a said link element; a said link element to another said link element; and/or a said link element to the tip element.

Preferably the actuator comprises a gear worm mechanism.

Further described is a method of controlling a device for grasping an object of any preceding claim, the method comprising: monitoring a mode of grasp of the object using one or more sensors disposed on a user, triggering an image capturing apparatus to acquire information about the object, inputting data from the sensors and image capturing device into a processor, activating the actuator of the device to control the device to move to a predefined grasp pattern based on the data input into the processor.

Preferably the mode of grasp of the object includes an opening mode, a pre-shaping mode and a closing mode.

Advantageously the information acquired by the image capturing device includes the shape of the object and a distance between the object and the device.

Preferably the predefined grasp pattern is selected from the group consisting of cylindrical grasp, lateral grasp, tip grasp, tripod grasp, spherical grasp, full-open and full-close.

According to a third aspect of the invention there is a myoelectric prosthetic hand controllable by a user comprising: one or more of the device of Claim 1; at least one electrode configured to be disposed on the user; an image capturing apparatus capable of capturing information about an object; and a processor operative to receive the information from the image capturing apparatus to provide for actuation of the device to move in a predetermined manner.

Preferably the at least one device moves in a predetermined manner which simulates the movement of a human finger.

A person of skill in the art will appreciate, in view of the foregoing, that the invention comprises a device to grasp an object and a control mechanism for controlling the device to avoid 'ejection' while maintaining a strong enough grasp on the object. The technical solution provided comprises an actuator to drive different elements or parts of the device. In some embodiments, the actuator comprises an electrical motor operable to drive a worm gear mechanism, operable to drive different parts of the device. The force provided by the actuator may be regarded as a primary force. One or more elastic mechanisms, in the form of springs, may be positioned between different parts of the device to provide a secondary force to collaborate with the primary force so as to minimize or eliminate 'ejection'.

Throughout this document, unless otherwise indicated to the contrary, the terms "comprising", "consisting of", and the like, are to be construed as non-exhaustive, or in other words, as meaning "including, but not limited to".

## BRIEF DESCRIPTION OF THE DRAWINGS

There now follows a description of various embodiments of the invention, with reference being made to the accompanying drawings, in which like reference numbers indicate like features, and wherein:

Figure 1 is a schematic view of an embodiment of a device of the invention.

Figure 2 is a schematic view showing forces acting on an embodiment of a device of the invention.

Figures 3 to 7 illustrate the movement of an embodiment of a device of the invention.

Figure 8 is a flowchart showing the steps involved in a method of operating a myoelectric prosthetic hand of the invention.

Figure 9 is a schematic flowchart of the apparatuses involved in carrying out the steps in Figure 8.

## EMBODIMENTS OF THE INVENTION

Although the various embodiments of the invention will be described with respect to a prosthetic hand device, this is for illustrative purposes only. The device is applicable to any prosthetic limbs. Moreover one of ordinary skill in the art will recognise that the various embodiments of the invention can be applied to any type of device configured for grasping objects. Reference to grasping include any manner of clutching, seizing and holding objects at a desired force.

In the following description of the invention, like numbers refer to like parts.

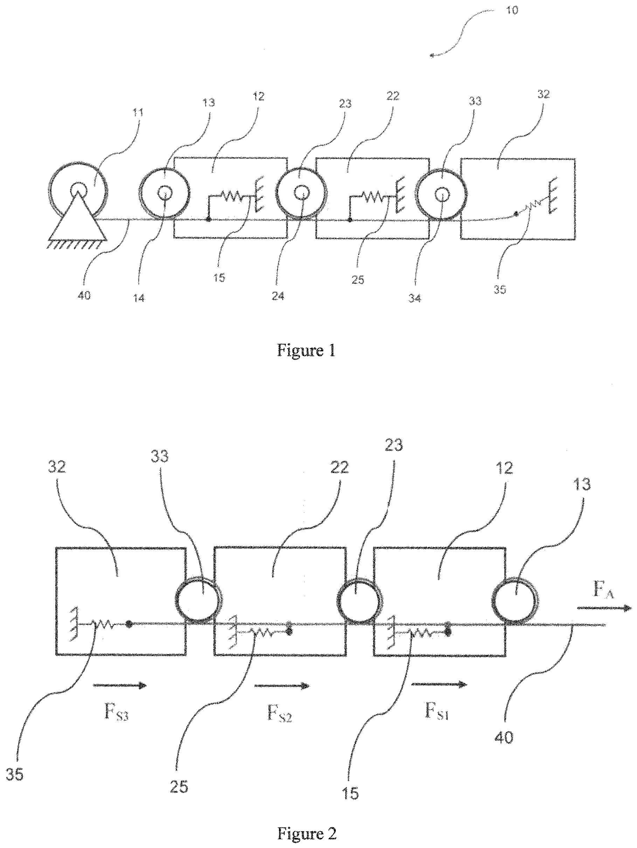

Figure 1 depicts an embodiment of the device 10 of the invention. The device 10 may be a prosthetic finger forming part of a prosthetic hand. The device 10 comprises an actuator 11. The actuator 11 can be any mechanism that provides an actuating force, such as a Shape Memory Alloy (SMA) or an Electroactive polymer, and may comprise an electrical motor arrange to drive one or more worm gear mechanism (not depicted). The worm gear mechanism comprises a gear ratio of at least 1:20, but may be in a range from 1:18 to 1:50. The electrical motor may be a stepper motor, an ultrasonic motor, DC motor, brushless motor, or any type of motor energized by and electrical source such as a battery.

Adjacent to the actuator 11 is a proximal element 12 comprising a pivot point in the form of a wheel 13 rotatable about an axle 14. One end of an elastic mechanism is attached to the proximal element 12. The elastic mechanism as illustrated is a spring 15 but any resilient material such as rubber, flexible-plastic could be used as long as the elastic coefficient of the material is known.

One end of a link element 22 is coupled to the proximal element 12. The link element 22 also comprises a wheel 23 rotatable about an axle 24; and one end of a spring 25 is attached to the link element 22. A tip element 32 is coupled to the other end of the link element 22. The tip element 32 is the furthest element from the actuator 10. Similarly, the tip element 32 comprises a wheel 33 rotatable about an axle 34; and one end of a spring 35 is attached to the tip element 32. Although Figure 1 only shows one link element 22 between the proximal element 12 and the tip element 32, it is to be appreciated there can be a plurality of link elements 22.

The worm gear mechanism is operable to achieve a "mechanical lock" wherein the system is powered in one direction only, i.e. from the actuator 11 to the elements of the device 10. In other words, the elements of the device 10 cannot apply power to the actuator 11. The worm gear mechanism will prevent the power that is generated from the link, from being transferred to the actuator, for example by pushing the link directly.

The wheels 13, 23 and 33 are connected by a cable 40 running around them and arranged to form a pulley system. Cable 40 can include any form of cord, link, rope or wire that can be used with the wheels to form a pulley system. A first end of the cable 40 is attached to an actuator 11. A primary force from the actuator 11 is transferred along the cable wire 40 to the proximal, link and tip elements 12, 22, 32.

The other end of the springs 15, 25 and 35 that is not connected to the element 12, 22 and 32, is connected to the cable 40. The springs 15 and 25 coupled to the proximal element 12 and link element 22 may be arranged or connected in parallel to the cable 40 to provide a secondary force to each of the proximal element 12 and link element 22 to reinforce the primary force so as to reduce or eliminate 'ejection'.

The elastic coefficient value of each of spring 15, 25 and 35 may be selected by the electrical power provided by the actuator 11. An example of how the elastic coefficient value could be selected for each spring is described as follows.

In a preferred embodiment wherein the device consists of three elements (i.e. a proximal element 12, a link element 22 and a tip element 32) the elastic coefficient of the spring 35 of the tip element 32 which is the furthest element of the device 10 from the actuator 11 will be selected first according to the technical specification of the actuator 11. In some embodiments the actuator 11 may be in the form of a small DC motor. The small DC motor has a stall torque (which is the maximum torque that can be applied to the shaft and cause the motor to stop rotating) of 0.3 to 1.6 Kg-cm (0.02941995 to 0.1569064N-m) at a rotational speed of 120-625 rpm. Taking the case of a motor with a stall torque of 0.3 Kg-cm transferring power to the worm gear mechanism and considering the efficiency of the transfer mechanism, the force that pulls along the cable is determined to be 10 N-cm. Accordingly, the spring coefficient of the tip element is being selected such that it does not provide an opposite force that exceeds 10 N-cm when it is in a fully extended state. Then the elastic coefficient of the spring 25 of link element 22 is selected to be less than or equal to that of the spring 35 of the tip element 32. Lastly, the elastic coefficient of the spring 15 of the proximal element 12 is selected to be less or equal to the link element 22.

In an alternative embodiment, without a pulley between the actuator 11 and the proximal element 12, the invention system will work if the elastic coefficients of the springs 15, 25 and 35 are selected to be from low to high, from tip element 32 to proximal element 12 accordingly.

Figure 2 illustrates a preferred embodiment of the invention in which four forces, $F_A$, $F_{S1}$, $F_{S2}$, and $F_{S3}$, represent the force of the actuator 11, the spring 15 of the proximal element 12, the spring 25 of the link element 22, and the spring 35 of the tip element 32 respectively. The only forces acting on the prosthetic finger come from the springs 15, 25 and 35. Summation of the spring forces is therefore equal to the force of the actuator 11 and is represented by the following equation:

$$F_A = F_{S1} + F_{S2} + F_{S3}$$

In this case, the three forces that apply to the device will be distributed from the actuation force according to their elastic coefficient and range.

The spring 34 of the tip element 32 is attached in series with the cable 40 wherein the terminal end of the cable 40 is connected to the end of the spring not attached to the tip element.

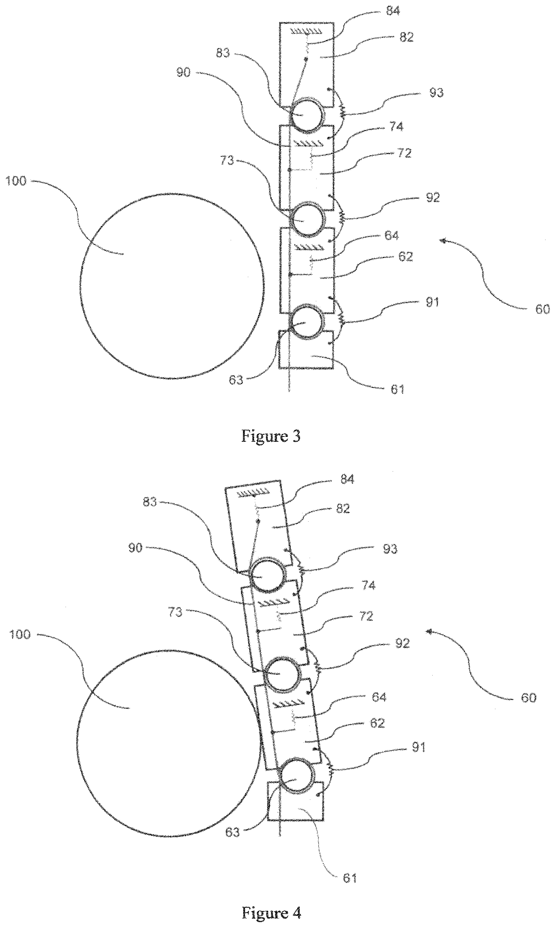

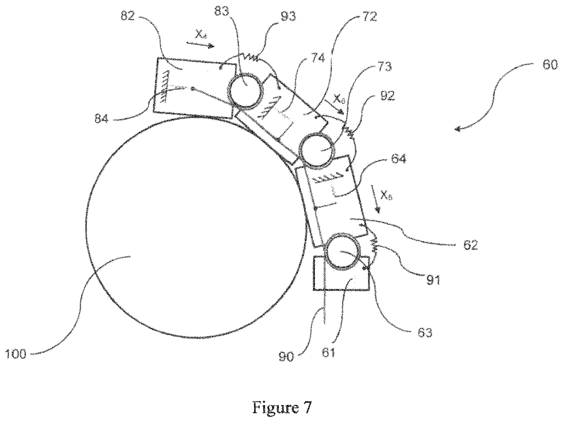

Figures 3 to 7 show the general movement of a device 60 when actuated to grasp an object 100. The device 60 has a base 61 forming a support that connects the prosthetic finger to the hand base.

Similar to the device 10 of Figure 1, device 60 has a proximal element 62, a link element 72 and a tip element 82. The device 60 further comprises wheels 63, 73 and 83. The wheels 63, 73 and 83 are connected by a cable 90 running around them. Springs 64, 74 and 84 are connected to the proximal element 62, the link element 72 and the tip element 82 respectively. The above description of device 10 applies to the device 60.

Device 60 further comprises resilient elements in the form of springs 91, 92, and 93. Spring 91 is coupled to the base 61 and the proximal element 62; spring 92 is coupled to the proximal element 62 and the link element 63; spring 93 is coupled to the link element 63 and the tip element 64. If there is more than one link element, a resilient element can be used to couple one link element to another. These resilient elements provide an extension force to the device 61, allowing the device 61 to increase its length if necessary to grasp an object.

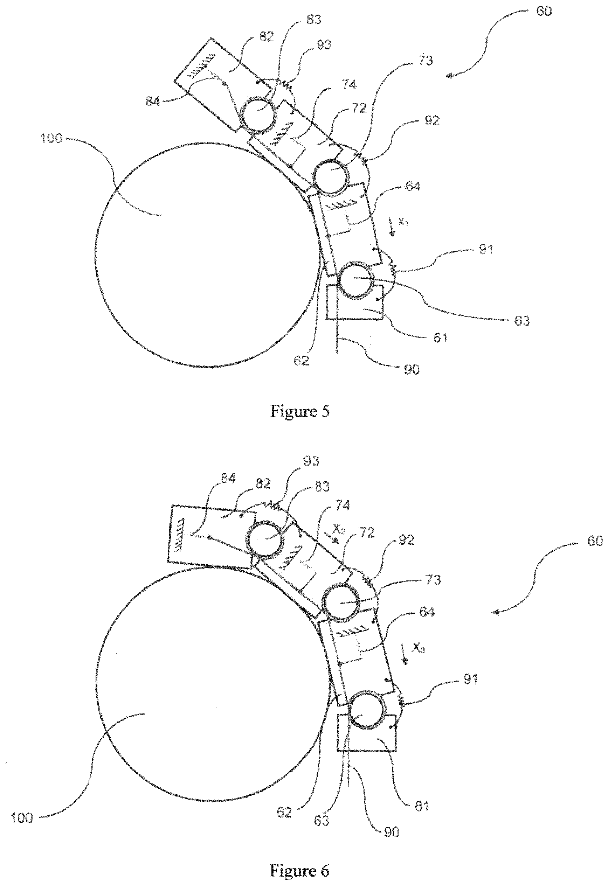

In Figures 2 to 7, a primary force (from an actuator not depicted) is first transmitted via the cable 90 to the proximal element 62 thus moving it until it contacts with an object 100 as shown in Figure 3. Next, the cable 90 is further pulled by the actuating force resulting in the movement of the link element 63 such that it contacts the object 100 as shown in Figure 4. If the link element 63 moves, then the spring 64 will move with the extended length of $X_1$ (see Figure 4 to 7) from the initial position. Next, the cable 90 is further pulled resulting in the movement of the tip element 82 until it contacts the object 100 as shown in Figure 5. This time, the spring 74 will move with the extended length of $X_3$ while the spring 64 will extend further with length of $X_2$. The extension of the springs will change as the device moves and changes in length. Figure 6 shows that all of the links are contacted with the object 100. If the primary force is continued to be applied to the cable 90, the cable 90 is allowed to move further by pulling the spring 84 to increase to length $X_4$ at the end of the device 60, resulting in further extension of spring 64 to length $X_5$ and spring 74 to length $X_6$. Consequently, this provides a positive force on each element of the device 60, thus preventing the ejection phenomenon as mentioned above.

Grasping Process

The invention is capable of providing for the formation of automatic preshaping grasp patterns.

The following is a description of the different stages of the grasping process in a prosthetic hand. While the object is being approached, the hand takes on a preshape. This refers to such a shape of the fingers that allows to put the hand close to or around the object. The process of changing hand posture from the previous hand posture to this preshape is called preshaping and can be seen as a preparation for grasping. The process of approaching the object as far as possible with this preshaped hand is referred to as pregrasping. Preshaping and pregrasping constitute the pregrasp. Subsequently, some or all fingers are closed until the grip is adequately tight. This closing process will be called finalising the grasp in this dissertation; the hand posture resulting from this is the final grasp. Depending on the object, there might furthermore be a trigger available, which refers to a fast movement with one or two fingers to use a certain functionality of the object, e.g. to push a button. A grasp type can be seen as a set of a preshape, a final grasp and possibly an available trigger. For some objects, the final grasp could be reached from a neutral hand position directly. However, for more complex objects like those including handles, a preshape is necessary to get fingers into the right position before finalising the grasp. If preshape and final grasp were automatically triggered without a break in between, patients using prostheses might not have enough time to find an appropriate hand position before finalising the grasp. Therefore, the usual grasping procedure with an active prosthesis includes both a pregrasp and a final grasp.

Prosthetic hand

The invention relates specifically to an externally powered prosthetic hand controlled by myoelectric signals, meaning it uses muscle signals in the user's residual limb to move. As mentioned earlier, the invention includes any prosthetic limb and is not limited to a prosthetic hand.

Electrodes are placed on the user's bare skin above two pre-selected muscle sites. When a user contracts these muscles, the electrodes pick up subtle changes in the electrical patterns and send these signals to a microprocessor which instructs the fingers to open and/or close.

Methods of attaching the prosthetic hand to the residual limb are known to the skilled person and the invention is not limited to any particular form of attachment. One example is the use of a custom made socket which fits the residual limb and to distribute the forces of the artificial limb across the area of the residual limp.

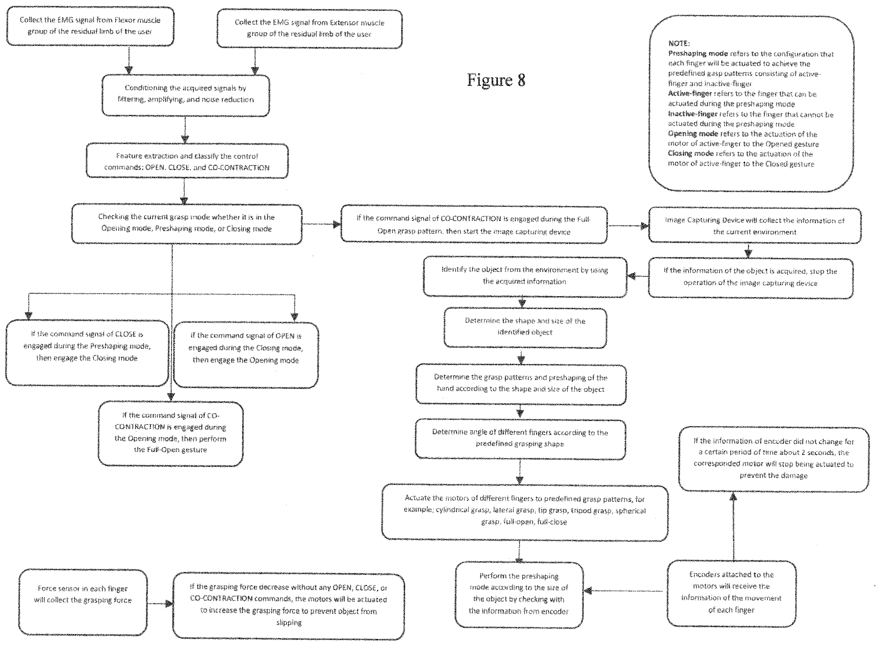

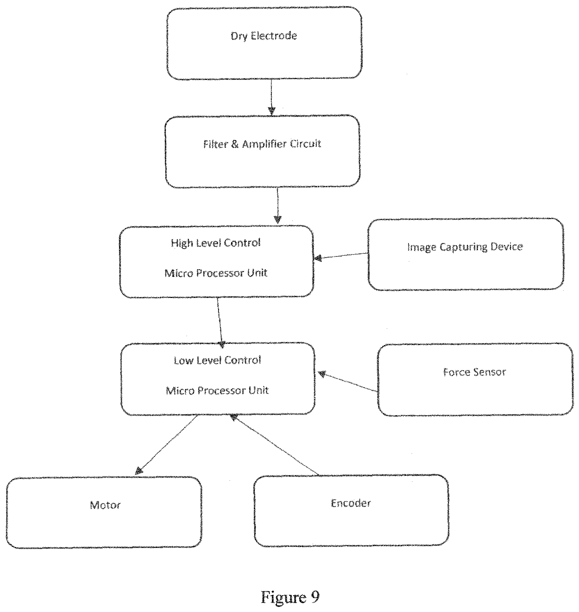

Referring to Figures 8 and 9, in the case of a prosthetic hand, biocompatible electrodes are implanted into different peripheral arm nerves of amputee user. Movement commands of the user are decoded from signals in the implanted electrodes and transmitted to the prosthetic hand, where they are translated into movements of the prosthetic hand. In an example, EMG signals from the flexor and extensor muscle groups of the residual limb of the user are collected when the user intentionally engage these muscles with the aim of grasping an object. These EMG signals are passed through a filter and amplifier circuit to condition them such as by filtering, amplifying and reducing noise. The filter and amplification may be implemented in hardware and/or software using methods known to the skilled person in the field of signal processing. For example in hardware, the electrical circuit of different types of filters could be designed to be a low-pass filter, a high-pass filter, or a notch filter at specific cut-off frequency to acquire the interested signal. Similarly, the amplifier can be designed to suit the particular electrical circuit. Sensors relay the conditioned signals to a high level control micro-processor which classify the control commands of the user. The control commands include:

- "Open"

- "Close"

- "Co-contraction"

The current grasp mode is checked to determine whether the prosthetic hand is in the

- Opening mode

- Preshaping mode or

- Closing mode.

Each control command is classified by extracting the EMG signal. For example, when the signal of a flexor muscle group is above a pre-determined threshold, the device will provide or issue the "Close" command. Similarly, when the signal of an extensor muscle group is above a pre-determined threshold, the device will provide or issue the "Open" command. The "Co-contraction" command is provided or issued when both signal of muscle group are over the set threshold.

The various grasp modes represent the different operational states of the prosthetic hand. Opening mode refers to the state where the hand is already opened or still in the opening process. Closing mode and Preshaping mode belong to the same operational state. However the closing mode refers to the state where the hand is already closed or still in the process of closing, and the preshaping mode refers to the state where the hand is already in the preshaping gesture or still in the preshaping process.

If the command signal of Co-contraction is engaged during the Full-Open grasp pattern (the Full-Open grasp pattern refers to the situation where every finger is fully extended to its limit), an image capturing device located on the prosthetic hand is activated to capture information about the environment. When the object has been detected by the image capturing device, the image capturing device is deactivated. The high level control micro-processor uses the captured information to identify the object from its environment, determining the shape and size of the object.

Next, the grasp pattern and preshaping of the hand is determined based on the shape and size of the object. The angle of each of the fingers of the prosthetic hand is determined once the predefined grasping shape has been established. The predefined grasp pattern is selected from the group consisting of cylindrical grasp, lateral grasp, tip grasp, tripod grasp, spherical grasp, full-open and full-close. Before performing the preshaping mode, the processor checks the information provided by encoders attached to the actuators regarding the movement of each finger. The preshaping mode will then be performed according to the information gathered regarding the object.

The preferred embodiment includes a force sensor on each finger to collect information about the grasping force. If the grasping force decreases without any Open, Close or Co-contraction commands, the actuator will be activated to increase the grasping force to prevent the object from slipping.

It should be appreciated by the person skilled in the art that variations and combinations of features described above, not being alternatives or substitutes, may be combined to form yet further embodiments falling within the intended scope of the invention.

The invention is advantageous because it is an imaging system that makes use of an image detection apparatus such as a stereo vision or camera to provide depth information of the captured image. This depth information is useful for classifying the shape of the object and the size of it. It is not possible and not practical for existing systems to capture such information (i.e. at best only providing a 2-dimensional information). The invention therefore provides several technical advantages over prior art systems.

**Claims**

1. A device (10) for grasping an object (100), the device comprising:

 an actuator (11);
 a proximal element (12) proximal to the actuator (11);
 a tip element (32) distal to the actuator (11);
 one or more link elements (22) between the proximal element (12) and the tip element (32) coupled by at least one pivot point connected by a cable (40); and
 an elastic mechanism;
 **characterized in that**
 the elastic mechanism comprises springs (15, 25, 35) and wherein each spring has a first end coupled to the cable (40) and a second end coupled to the proximal, link or tip elements; and wherein
 the actuator (11) provides a primary force on the proximal and tip elements;

the proximal and tip elements are coupled by the pivot point connected by the cable to the actuator (11), and the elastic mechanism acts on the cable (40) to provide a secondary force which displaces the proximal and tip elements towards the object (100).

2. The device of Claim 1 wherein the spring (15) coupled to the proximal element (12) is connected parallel to the cable (40).

3. The device of Claim 1 wherein the spring (25) coupled to a said link element (22) is connected parallel to the cable (40).

4. The device of any of Claims 2 or 3 comprising one or more resilient elements (91, 92, 93) capable of coupling:

a support base of the device to the proximal element (12);
the proximal element (12) to a said link element (22);
a said link element to another said link element; and/or
a said link element to the tip element (32).

5. The device of any preceding claim wherein the actuator (11) comprises a gear worm mechanism.

6. A myoelectric prosthetic hand controllable by a user **characterized in that** the prosthetic hand comprises:

one or more of the device (10) of Claim 1;
at least one electrode configured to be disposed on the user;
an image capturing apparatus capable of capturing information about an object; and
a processor operative to receive the information from the image capturing apparatus to provide for actuation of the device to move in a predetermined manner.

7. The prosthetic system of Claim 6 wherein the at least one device moves in a predetermined manner which simulates the movement of a human finger.

**Patentansprüche**

1. Vorrichtung (10) zum Greifen eines Objekts (100), wobei die Vorrichtung Folgendes umfasst:

einen Aktuator (11);
ein proximales Element (12), das proximal zum Aktuator (11) liegt;

ein Spitzenelement (32), das distal zum Aktuator (11) liegt;
ein oder mehrere Verbindungselemente (22) zwischen dem proximalen Element (12) und dem Spitzenelement (32), die durch mindestens einen Drehpunkt gekoppelt sind, der durch ein Kabel (40) verbunden ist; und
einen elastischen Mechanismus;
**dadurch gekennzeichnet, dass**
der elastische Mechanismus Federn (15, 25, 35) umfasst, und wobei jede Feder ein erstes Ende, das mit dem Kabel (40) gekoppelt ist, und ein zweites Ende aufweist, das mit dem proximalen, dem distalen oder dem Spitzenelement gekoppelt ist; und
wobei
der Aktuator (11) dem proximalen und dem Spitzenelement eine primäre Kraft bereitstellt;
das proximale und das Spitzenelement durch den Drehpunkt, der durch das Kabel mit dem Aktuator (11) verbunden ist, gekoppelt sind, und der elastische Mechanismus auf das Kabel (40) wirkt, um eine sekundäre Kraft bereitzustellen, die das proximale und das Spitzenelement in Richtung des Objekts (100) verschiebt.

2. Vorrichtung nach Anspruch 1, wobei die Feder (15), die mit dem proximalen Element (12) gekoppelt ist, parallel mit dem Kabel (40) verbunden ist.

3. Vorrichtung nach Anspruch 1, wobei die Feder (25), die mit einem Verbindungselement (22) gekoppelt ist, parallel mit dem Kabel (40) verbunden ist.

4. Vorrichtung nach einem der Ansprüche 2 oder 3, die ein oder mehrere federnde Elemente (91, 92, 93) umfasst, die Folgendes koppeln können:

eine Trägerbasis der Vorrichtung mit dem proximalen Element (12);
das proximale Element (12) mit einem Verbindungselement (22);
ein Verbindungselement mit einem anderen Verbindungselement; und/oder
ein Verbindungselement mit dem Spitzenelement (32).

5. Vorrichtung nach einem der vorhergehenden Ansprüche, wobei der Aktuator (11) einen Schneckengetriebemechanismus umfasst.

6. Myoelektrische Handprothese, die von einem Benutzer steuerbar ist, **dadurch gekennzeichnet, dass** die Handprothese Folgendes umfasst:

eine oder mehrere der Vorrichtung (10) nach Anspruch 1;
mindestens eine Elektrode, die dazu konfiguriert ist, an dem Benutzer angeordnet zu werden; eine Bilderfassungsvorrichtung, die Informationen über ein Objekt erfassen kann; und einen Prozessor, der funktionsfähig ist, die Informationen von der Bilderfassungsvorrichtung zu empfangen, um das Betätigen der Vorrichtung bereitzustellen, damit diese sich in einer vorbestimmten Weise bewegt.

**7.** Prothesensystem nach Anspruch 6, wobei sich die mindestens eine Vorrichtung in einer vorbestimmten Weise bewegt, die die Bewegung eines menschlichen Fingers simuliert.

## Revendications

**1.** Dispositif (10) pour saisir un objet (100), le dispositif comprenant :

un actionneur (11) ;
un élément proximal (12) proximal par rapport à l'actionneur (11) ;
un élément bout (32) distal par rapport à l'actionneur (11) ;
un ou plusieurs éléments de liaison (22) entre l'élément proximal (12) et l'élément bout (32) accouplés par au moins un point pivot raccordé par un câble (40) ; et
un mécanisme élastique ;
**caractérisé en ce que**
le mécanisme élastique comprend des ressorts (15, 25, 35) et dans lequel chaque ressort a une première extrémité accouplée au câble (40) et une deuxième extrémité accouplée aux éléments proximal, de liaison ou bout ; et
dans lequel
l'actionneur (11) fournit une force primaire sur les éléments proximal et bout ;
les éléments proximal et bout sont accouplés par le point pivot raccordé par le câble à l'actionneur (11), et le mécanisme élastique agit sur le câble (40) pour fournir une force secondaire qui déplace les éléments proximal et bout vers l'objet (100).

**2.** Dispositif selon la revendication 1 dans lequel le ressort (15) accouplé à l'élément proximal (12) est raccordé parallèlement au câble (40).

**3.** Dispositif selon la revendication 1 dans lequel le ressort (25) accouplé à l'un desdits éléments de liaison (22) est raccordé parallèlement au câble (40).

**4.** Dispositif selon l'une quelconque des revendications 2 ou 3 comprenant un ou plusieurs éléments résilients (91, 92, 93) capables d'accoupler :

une base support du dispositif à l'élément proximal (12) ;
l'élément proximal (12) à l'un desdits éléments de liaison (22) ;
l'un desdits éléments de liaison à un autre desdits éléments de liaison ; et/ou
l'un desdits éléments de liaison à l'élément bout (32).

**5.** Dispositif selon une quelconque revendication précédente dans lequel l'actionneur (11) comprend un mécanisme à vis sans fin.

**6.** Main prothétique myoélectrique commandable par un utilisateur **caractérisée en ce que** la main prothétique comprend :

un ou plusieurs dispositifs (10) selon la revendication 1 ;
au moins une électrode configurée pour être disposée sur l'utilisateur ;
un appareil de capture d'image capable de capturer des informations sur un objet ; et
un processeur servant à recevoir les informations à partir de l'appareil de capture d'image pour assurer un actionnement du dispositif pour qu'il bouge d'une manière prédéterminée.

**7.** Système prothétique selon la revendication 6 dans lequel l'au moins un dispositif bouge d'une manière prédéterminée qui simule le mouvement d'un doigt humain.

Figure 1

Figure 2

Figure 3

Figure 4

Figure 5

Figure 6

Figure 7

## Figure 8

Collect the EMG signal from Flexor muscle group of the residual limb of the user

Collect the EMG signal from Extensor muscle group of the residual limb of the user

NOTE:
**Preshaping mode** refers to the configuration that each finger will be actuated to achieve the predefined gasp patterns consisting of active-finger and inactive-finger
**Active-finger** refers to the finger that can be actuated during the preshaping mode
**Inactive-finger** refers to the finger that cannot be actuated during the preshaping mode
**Opening mode** refers to the actuation of the motor of active-finger to the Opened gesture
**Closing mode** refers to the actuation of the motor of active-finger to the Closed gesture

Conditioning the acquired signals by filtering, amplifying, and noise reduction

Feature extraction and classify the control commands: OPEN, CLOSE, and CO-CONTRACTION

Checking the current grasp mode whether it is in the Opening mode, Preshaping mode, or Closing mode

If the command signal of CO-CONTRACTION is engaged during the Full-Open grasp pattern, then start the image capturing device

Image Capturing Device will collect the information of the current environment

Identify the object from the environment by using the acquired information

If the information of the object is acquired, stop the operation of the image capturing device

If the command signal of CLOSE is engaged during the Preshaping mode, then engage the Closing mode

If the command signal of OPEN is engaged during the Closing mode, then engage the Opening mode

Determine the shape and size of the identified object

If the command signal of CO-CONTRACTION is engaged during the Opening mode, then perform the Full-Open gesture

Determine the grasp patterns and preshaping of the hand according to the shape and size of the object

Determine angle of different fingers according to the predefined grasping shape

If the information of encoder did not change for a certain period of time about 2 seconds, the corresponded motor will stop being actuated to prevent the damage

Actuate the motors of different fingers to predefined grasp patterns, for example: cylindrical grasp, lateral grasp, tip grasp, tripod grasp, spherical grasp, full-open, full-close

Force sensor in each finger will collect the grasping force

If the grasping force decrease without any OPEN, CLOSE, or CO-CONTRACTION commands, the motors will be actuated to increase the grasping force to prevent object from slipping

Perform the preshaping mode according to the size of the object by checking with the information from encoder

Encoders attached to the motors will receive the information of the movement of each finger

Figure 9

**REFERENCES CITED IN THE DESCRIPTION**

*This list of references cited by the applicant is for the reader's convenience only. It does not form part of the European patent document. Even though great care has been taken in compiling the references, errors or omissions cannot be excluded and the EPO disclaims all liability in this regard.*

**Patent documents cited in the description**

- US 6896704 B1
- CN 105583839 A
- US 2006145495 A1